# EUROPEAN PATENT APPLICATION

(11) **EP 4 706 686 A1**
(43) Date of publication of application: **11.03.2026**
(21) Application number: 24819613.1
(22) Date of filing: 07.06.2024
(51) Int. Cl.: A61K 48/00, A61K 31/713, A61P 27/02

(54) **PHARMACEUTICAL COMPOSITION COMPRISING SCLEROSTIN INHIBITOR AS ACTIVE INGREDIENT FOR PREVENTION OR TREATMENT OF DIABETIC EYE DISEASE**

(30) Priority: 07.06.2023 KR 20230073048; 05.06.2024 KR 20240073989
(71) Applicant: Chung Ang University Industry Academic Cooperation Foundation, Seoul 06974 (KR)
(72) Inventor: KIM, Jee Taek, Seoul 06001 (KR)
(74) Representative: BCKIP Part mbB
(86) International application number: PCT/KR2024/007814
(87) International publication number: WO 2024/253462

(57) **Abstract**

The present invention relates to a pharmaceutical composition comprising a sclerostin inhibitor as an active ingredient for the prevention or treatment of diabetic eye disease, wherein the composition, by inhibiting sclerostin, not only reduces the expression levels of inflammatory cytokines significantly even under conditions of VEGF treatment and hyperglycemia, thereby exhibiting an excellent anti-inflammatory activity, but also is shown to have a significant angiogenic effect due to reduced expression of vascular adhesion markers and vascular endothelial growth factors, thereby inhibiting the principal characteristic of diabetic eye disease, the inflammatory response, and microvascular complications. In addition, the composition reduces oxidative stress caused by diabetes and does not induce cell death and thus can be useful as a preparation for the prevention or treatment of diabetic eye disease.

## Description

### THECHNICAL FIELD

The present invention relates to a pharmaceutical composition comprising sclerostin inhibitor as active ingredient for prevention or treatment of diabetic eye disease.

This application claims priority based on Korean Patent Application No. 10-2023-0073048 filed on June 7, 2023, and the entire contents of the specification and figures disclosed in the application are incorporated herein by reference.

This application claims priority based on Korean Patent Application No. 10-2024-0073989 filed on June 5, 2024, and the entire contents of the specification and figures disclosed in the application are incorporated herein by reference.

### BACKGROUND OF THE INVENTION

Diabetes mellitus is a systemic, non-infectious chronic disease characterized by abnormally elevated blood glucose(sugar) levels that persist over a long period, representing a classic metabolic disorder. Insulin, produced by the beta cells of the pancreas, plays a central role in the metabolism of glucose produced from the digestion and absorption of carbohydrates. It is a hormone that regulates blood glucose levels within a specific range to prevent hyperglycemia.

Diabetes results either from an absolute or relative deficiency in insulin production caused by the destruction of pancreatic beta cells through autoimmune mechanisms, or from a reduction in insulin's effective action (insulin resistance) in target organs despite insulin secretion. Consequently, blood glucose levels remain persistently elevated, preventing the body's metabolic functions-including glucose metabolism-from functioning properly. Diabetic patients cannot utilize glucose, the body's primary energy source, leading to a disruption of bodily balance. This imbalance causes various complications and poses a threat to life.

Symptoms of diabetes comprise chronic inflammation. Chronic inflammation involves a mild elevation of inflammatory cytokines such as TNF-α, IL-1β, and IL-6, triggered by endogenous or exogenous factors. In chronic inflammation, macrophages are a major source of inflammatory cytokines, and human macrophages have been shown to produce more pro-inflammatory cytokines in response to hyperglycemia. It has been demonstrated that chronic inflammation, specifically asymptomatic persistent systemic inflammation, promotes vascular damage.

Furthermore, hyperglycemia increases markers of inflammation and oxidative stress through a vicious cycle, which is known to play a crucial role in the development of diabetic complications. Beyond hyperglycemia itself, it induces numerous pathophysiological pathways associated with the onset of diabetic complications, including the polyol pathway, oxidative stress, non-enzymatic glycation, and activation of protein kinase C.

The most common complications of diabetes comprise diabetic retinopathy, diabetic macular edema, glaucoma, and cataracts. Diabetic retinopathy (DR), the most common microvascular outcome of diabetes, is a leading cause of vision loss worldwide, affecting an estimated 93 million people, with 21 million suffering from diabetic macular edema.

Thus, while the need for research on developing treatments for diabetic eye diseases is increasing, little is known about formulations utilizing sclerostin for treating diabetic eye diseases.

### DETAILED DESCRIPTION OF THE INVENTION

### TECHNICAL PROBLEM

The object of the present invention is to provide a pharmaceutical composition comprising a sclerostin inhibitor as an active ingredient for prevention or treatment of diabetic eye disease.

Another object of the present invention is to provide a food composition comprising a sclerostin inhibitor as an active ingredient for prevention or improvement of diabetic eye disease.

Yet another object of the present invention is to provide a kit comprising a sclerostin inhibitor and instructions for prevention or treatment of diabetic eye disease.

However, the technical problem that the present invention is intended to achieve is not limited to the problems mentioned above, and other problems not mentioned can be clearly understood by one of ordinary skill in the art to which the present invention pertains from the description below.

### TECHNICAL SOLUTION

The present invention provides a pharmaceutical composition comprising a sclerostin inhibitor as an active ingredient for prevention or treatment of diabetic eye disease.

In one embodiment of the present invention, wherein the inhibitor may be at least one of an expression inhibitor or an activity inhibitor, but is not limited thereto.

In one embodiment of the present invention, wherein the expression inhibitor or the activity inhibitor may be at least one selected from the group consisting of small interfering RNA (siRNA), short hairpin RNA (shRNA), micro RNA (miRNA), antisense oligonucleotides (ASO), Clustered Reg ularly Interspaced Short Palindromic Repeats (CRISPR/Cas), natural products, proteins, peptidomimetics, antibodies, exosomes, and compounds, but is not limited thereto.

In one embodiment of the present invention, wherein the diabetic eye disease may be any one or more selected from the group consisting of diabetic retinopathy, diabetic macular edema, glaucoma, and cataract, but is not limited thereto.

In one embodiment of the present invention, wherein the composition may any one formulation selected from the group consisting of suspensions, gels, ointments, injectable solutions, sprays, and eye drops., but is not limited thereto.

In one embodiment of the present invention, wherein the composition may inhibit inflammatory responses and microvascular complications, but is not limited thereto.

In one embodiment of the present invention, wherein the composition may be characterized by comprising one or more of the following, but is not limited thereto:
inhibition of inflammatory cytokines within retinal cells;
inhibition of angiogenesis;
inhibition of vascular endothelial growth factor and vascular adhesion markers; and
reduction of oxidative stress.

The present invention provides a food composition comprising a sclerostin inhibitor as an active ingredient for prevention or improvement of diabetic eye disease.

In one embodiment of the present invention, wherein the food may be a health functional food, but is not limited thereto.

The present invention provides a kit comprising a sclerostin inhibitor and instructions for prevention or treatment of diabetic eye disease.

In addition, the present invention provides a method for prevention or treatment of diabetic eye disease, comprising the step of administrating a composition comprising a sclerostin inhibitor as an active ingredient in a pharmaceutically effective amount to a subject in need thereof.

In addition, the present invention provides an use of a composition comprising a sclerostin inhibitor as an active ingredient for the prevention or treatment of diabetic eye diseases.

In addition, the present invention provides an use of a composition comprising a sclerostin inhibitor as an active ingredient for preparing an agent for prevention or treatment of diabetic eye disease.

### ADVANTAGEOUS EFFECT

According to a pharmaceutical composition comprising a sclerostin inhibitor as an active ingredient for prevention or treatment of diabetic eye disease, inhibiting sclerostin significantly reduces the expression levels of inflammatory cytokines even under hyperglycemic and VEGF-treated conditions, demonstrating not only excellent anti-inflammatory activity but also a marked anti-angiogenic effect due to decreased expression of vascular endothelial growth factor and vascular adhesion markers, demonstrating a significant anti-angiogenic effect. This confirms its ability to suppress the inflammatory response and microvascular complications, which are the most prominent features of diabetic eye disease. Furthermore, it reduces oxidative stress induced by diabetes and does not induce cell death, making it a useful agent for the prevention or treatment of diabetic eye disease.

### BRIEF DESCRIPTION OF THE DRAWINGS

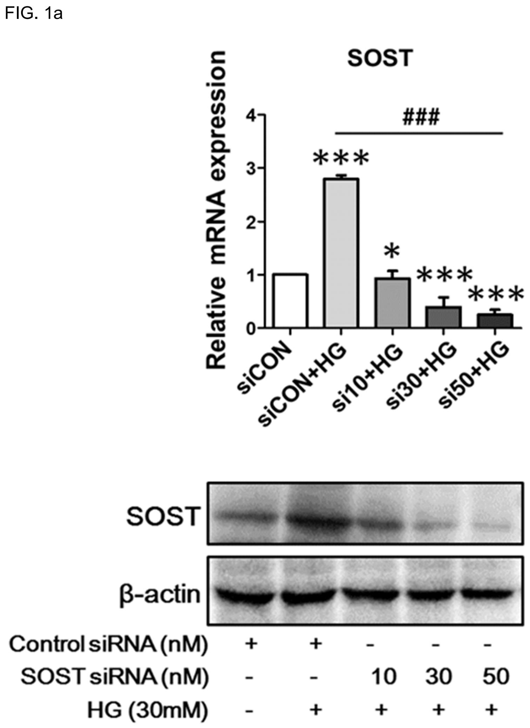

FIG. 1a shows the results of mRNA expression and Western blot analysis for SOST expression following siRNA transfection at various concentrations (10, 30, and 50 nM siRNA) labeled as si10, si30, and si50 under HG treatment.

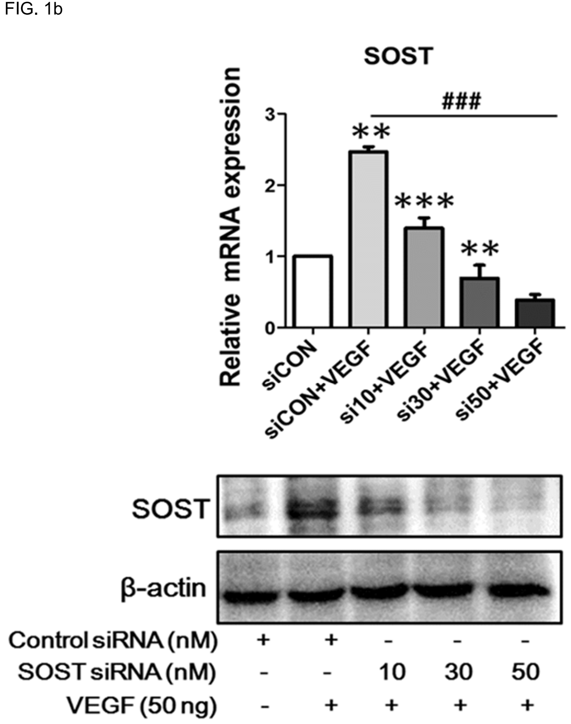

FIG. 1b shows the results of mRNA expression and Western blot analysis of SOST expression after siRNA transfection with different concentrations (10, 30, 50 nM siRNA) under VEGF treatment(*P < 0.05, **P < 0.01, ***P < 0.001 control, #P < 0.05, ##P < 0.01, ##P < 0.001.01, ##P < 0.001).

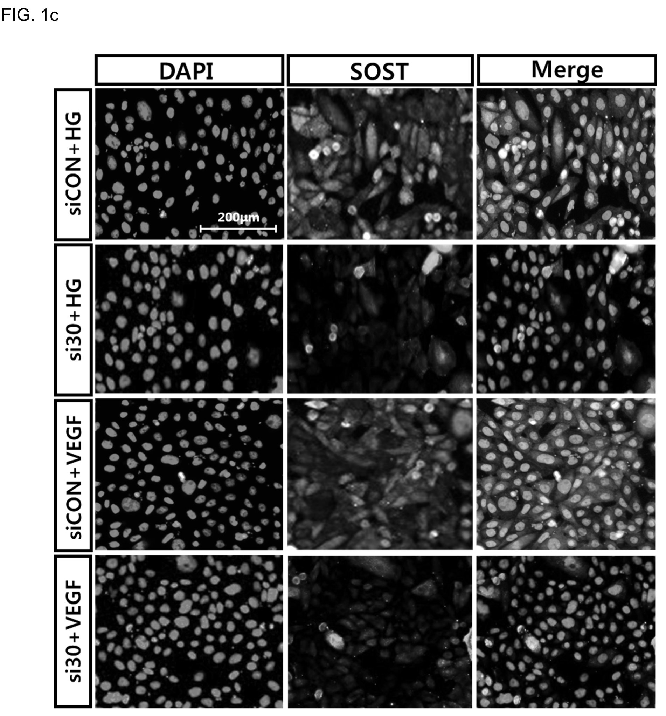

FIG. 1c shows the results of immunofluorescence analysis of SOST expression under HG and VEGF treatment.

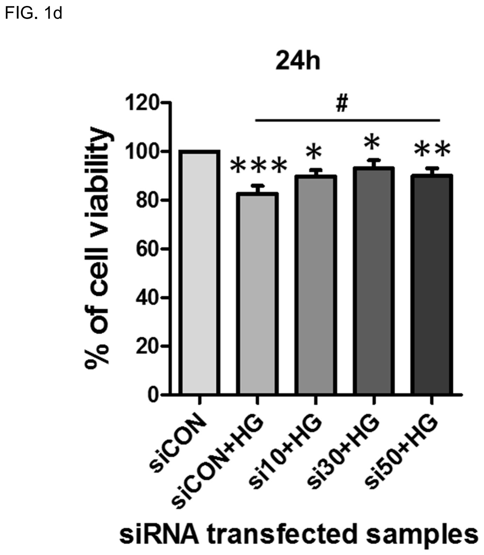

FIG. 1d shows the results of analyzing HREC viability using MTT after transfecting siRNA at various concentrations (10 nM, 30 nM, and 50 nM siRNA) under hyperglycemic conditions.

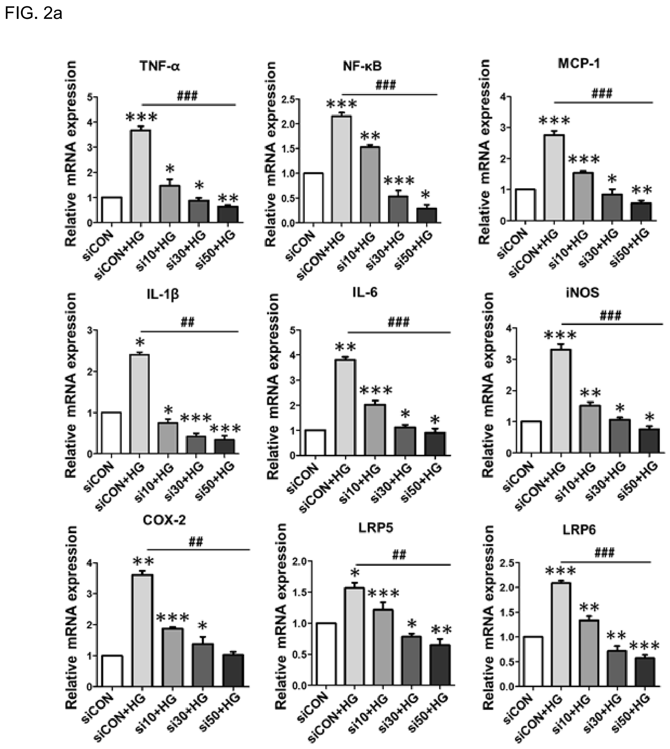

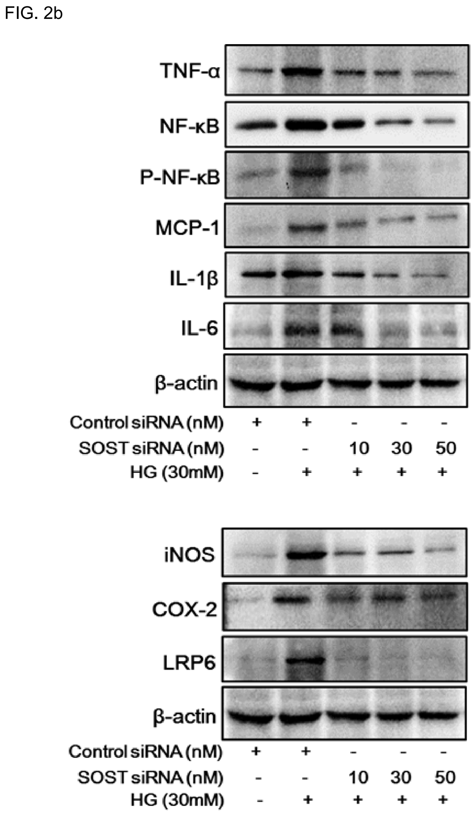

FIG. 2a and FIG. 2b show the results of analyzing changes in mRNA levels and protein expression levels of pro-inflammatory cytokine gene markers following SOST suppression respectively. Under hyperglycemic conditions, pro-inflammatory cytokines exhibit high protein expression, while following SOST knockdown, they show a tendency toward low expression.

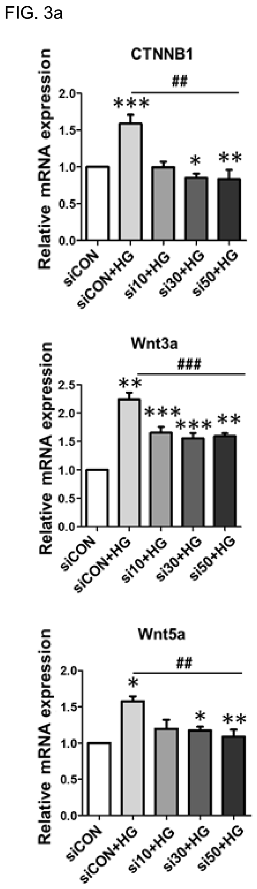

FIG. 3a shows the mRNA levels of the Wnt/β-catenin gene marker.

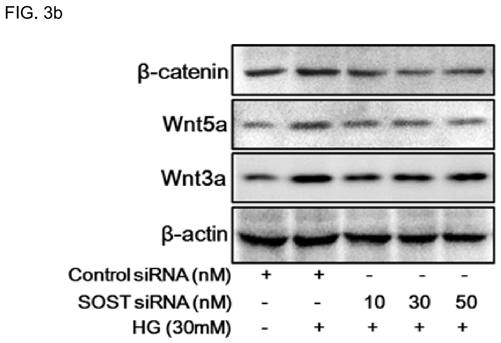

FIG. 3b shows the protein expression levels of Wnt/β-catenin after glucose treatment.

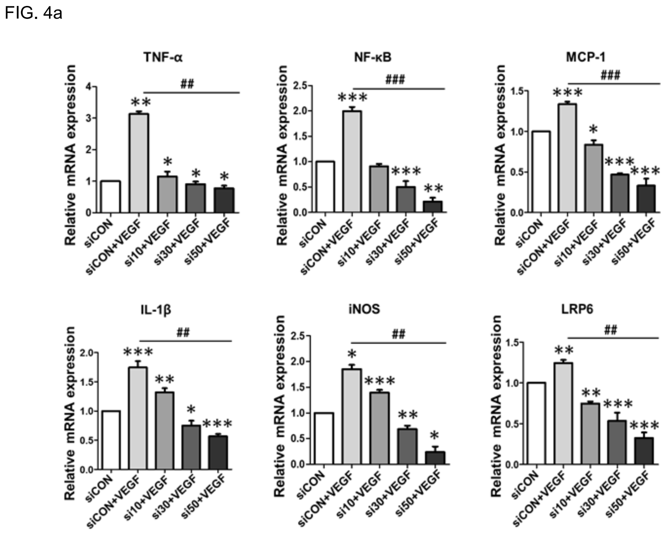

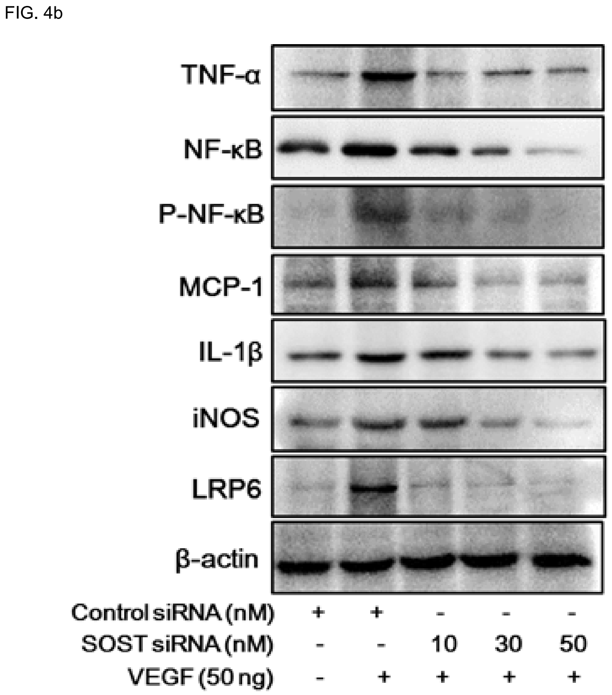

FIG. 4a and FIG. 4b show the high expression of pro-inflammatory genes after VEGF treatment and the low expression observed after sclerostin/SOST knockdown. FIG. 4a shows the mRNA levels of pro-inflammatory gene markers. FIG. 4b shows the protein expression levels of pro-inflammatory chemokines and cytokines after VEGF treatment.

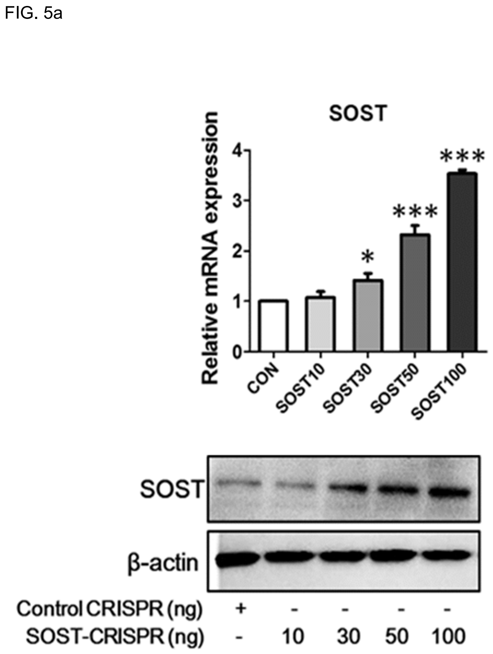

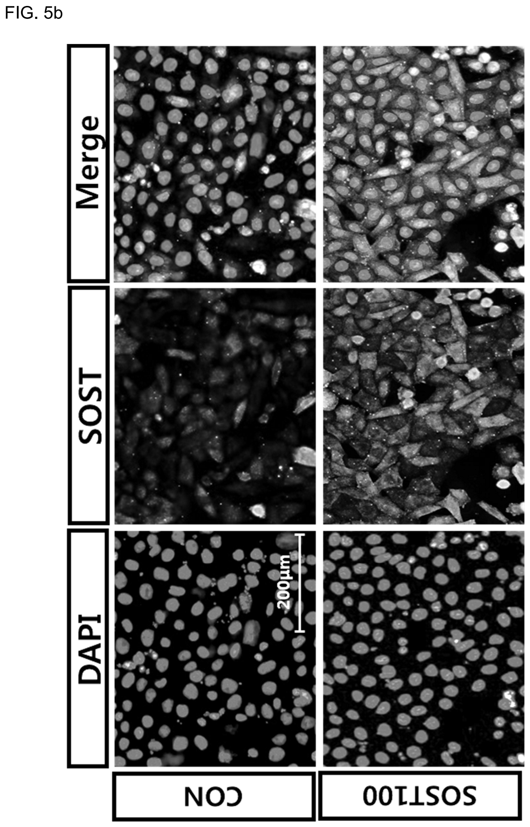

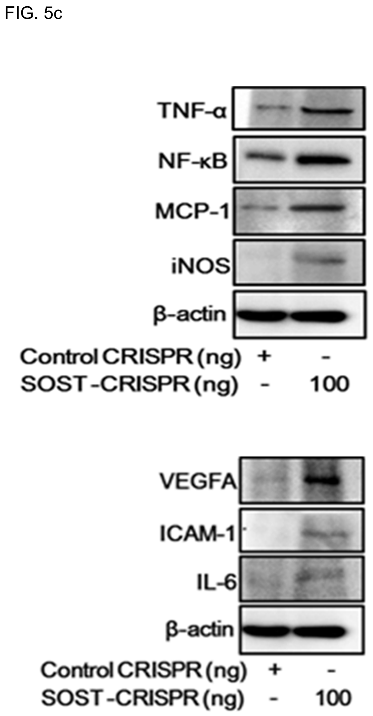

FIG. 5a to 5c present the results of analyzing SOST overexpression in HREC following CRISPR-SOST knockin. FIG. 5a shows high expression levels of SOST mRNA and protein. FIG. 5b presents the immunofluorescence analysis results for SOST overexpression. FIG. 5c demonstrates high expression levels of pro-inflammatory proteins following CRISPR-SOST knockin.

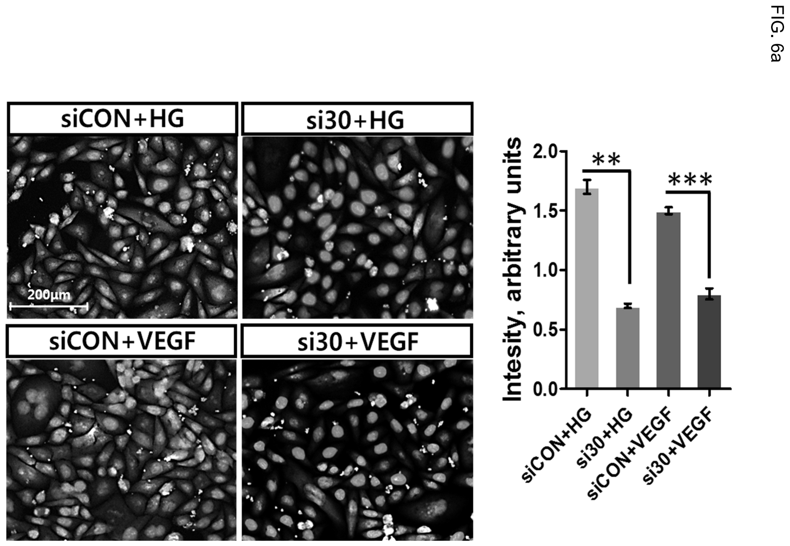

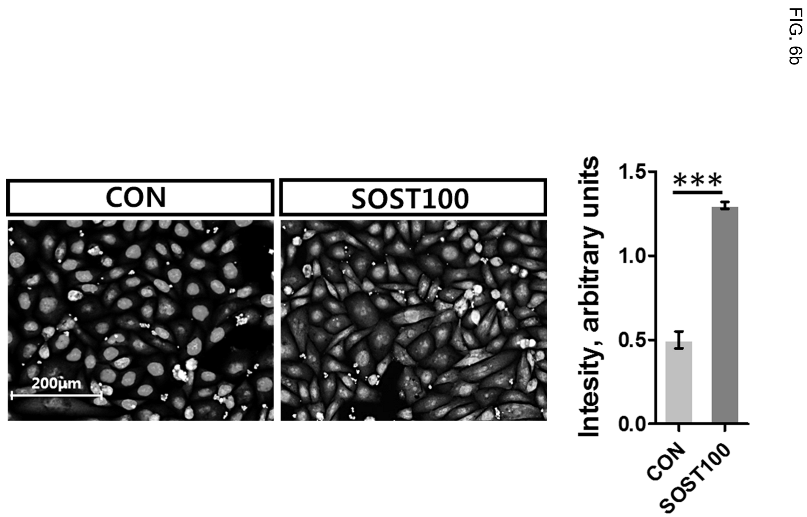

FIG. 6a and FIG. 6b show the results of measuring the reduction of oxidative stress in HRECs by SOST knockdown. FIG. 6a shows the high oxidative stress observed after HG and VEGF treatment and the low oxidative stress observed after SOST knockdown. FIG. 6b shows the results of measuring oxidative stress in HRECs following CRISPR-SOST knockin.

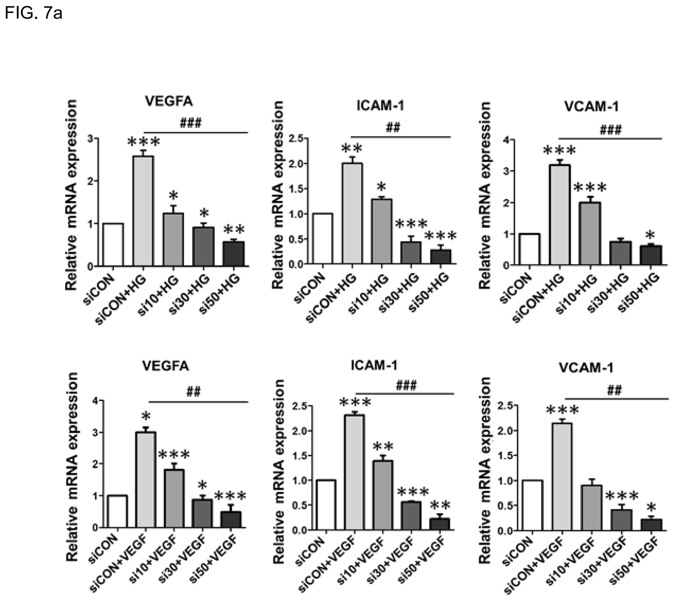

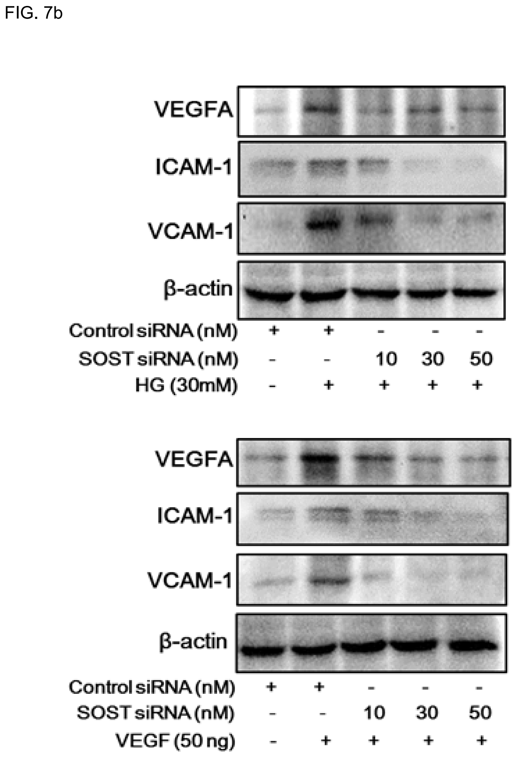

FIG. 7a and FIG.7b show the results of analyzing the expression levels of vascular endothelial growth factor A (VEGFA) and the blood adhesion molecules ICAM-1 and VCAM-1 in HREC cells transfected with SOST siRNA. FIG. 7a shows the mRNA and protein expression levels of VEGFA, ICAM-1, and VCAM-1 after HG treatment. FIG. 7b shows the mRNA and protein expression levels of VEGFA, ICAM-1, and VCAM-1 after VEGF treatment.

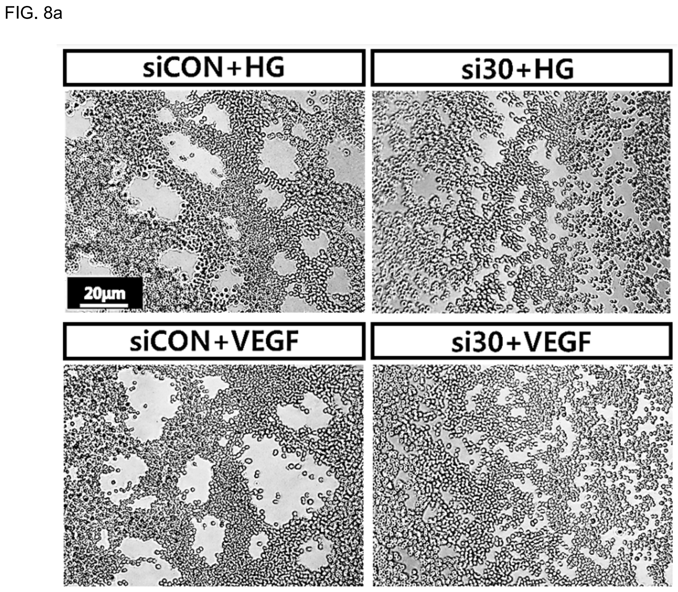

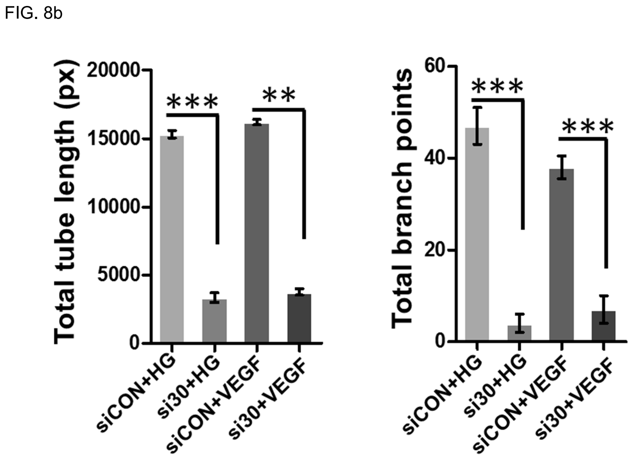

FIG.8a and FIG. 8b show the results of analyzing angiogenesis activity when SOST was silenced. FIG. 8a shows phase-contrast images of tube formation in HREC undergoing HG and VEGF treatment. FIG. 8b presents the results analyzed using ImageJ densitometry for total tube length (pixels, px) and the number of branching points in tube formation.

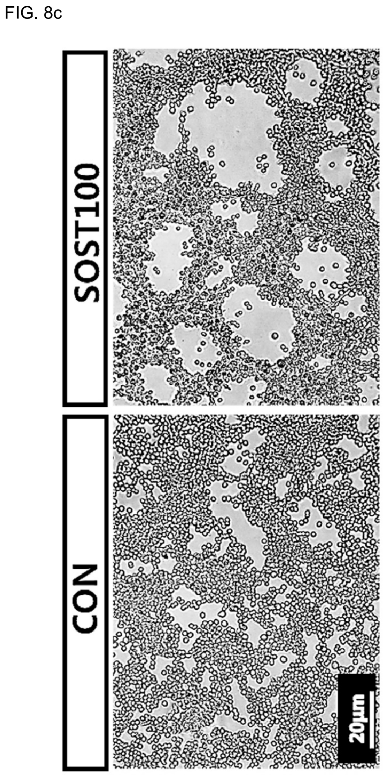

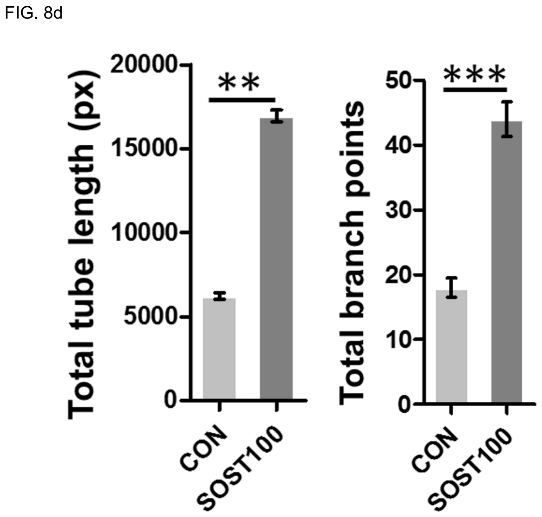

FIG. 8c and FIG. 8d show the results of analyzing angiogenesis activity when SOST was overexpressed. FIG. 8c shows the phase-contrast image of tube formation after CRISPR-SOST knockin. FIG. 8d shows the results of analyzing the total tube length (pixels, px) and the number of branching points in tube formation using ImageJ densitometry. Compared to siRNA-transfected samples, increased tube formation was observed in siCON + HG, siCON + VEGF, and SOST100 samples.

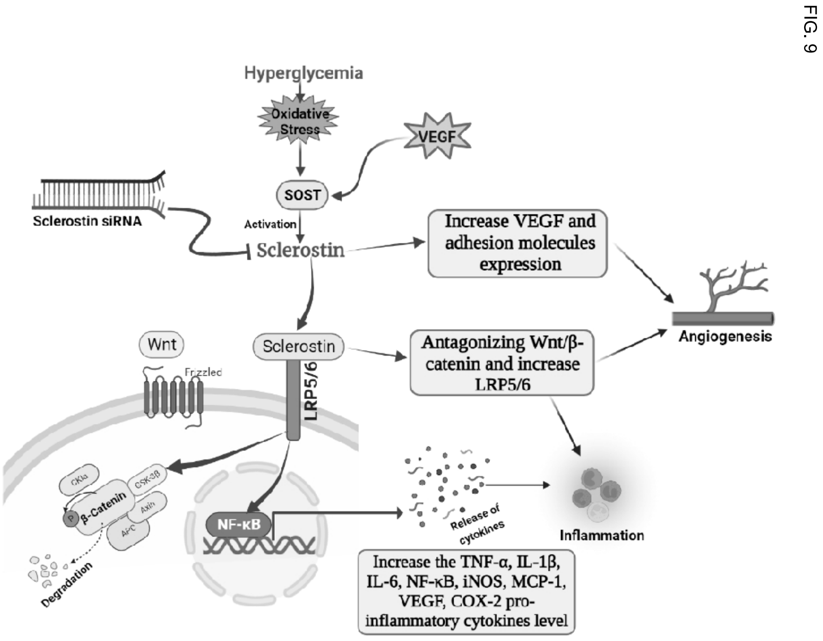

FIG. 9 is a schematic diagram illustrating the role of sclerostin in activating angiogenesis and endothelial inflammation via the NF-κB pathway under hyperglycemic conditions in HREC, the antagonism of Wnt/β-catenin signaling through binding to (LRP) 5/6, and the inhibitory effect of sclerostin-siRNA on the inflammatory response.

### BEST MODE FOR CARRYING OUT THE INVENTION

The present invention provides a pharmaceutical composition comprising a sclerostin inhibitor as an active ingredient for prevention or treatment of diabetic eye disease.

Since sclerostin is expressed by the SOST gene, the sclerostin inhibitor in the present invention may comprise an SOST expression inhibitor. Accordingly, the present invention provides a pharmaceutical composition comprising an SOST expression inhibitor as an active ingredient for prevention or treatment of diabetic eye disease.

In the present invention, the "sclerostin inhibitor" may comprise both a sclerostin expression inhibitor and a sclerostin activity inhibitor. Accordingly, in one embodiment of the present invention, wherein the inhibitor may be at least one of an expression inhibitor or an activity inhibitor, but is not limited thereto.

In the present invention, the "expression inhibition" may also comprise expression reduction, in addition to inhibition of expression. The "expression reduction" refers to any act of reducing the expression of a gene, and specifically may be achieved by introducing a variation such as knockout, knockdown, gene interference, or deletion, duplication, inversion, or replacement in the sequence of the gene DNA, and comprise all cases in which expression is reduced by all methods exemplarily proposed in the present invention.

In the present invention, the "activity inhibition" may also comprise activity reduction, in addition to inhibition of activity. The "activity reduction" refers to inhibition of one or more functions caused by sclerostin, and the function may be related to the occurrence of diabetic eye diseases, the progression or aggravation of the symptoms of diabetic eye diseases, or the increase in the progression speed of diabetic eye diseases, or may be a function related to the occurrence or worsening of signs of diabetic eye diseases, but is not limited thereto.

In addition, in the present invention, it was confirmed that when sclerostin was overexpressed, diabetic eye diseases were induced or the symptoms were aggravated, and when the SOST gene was knockdown, the above symptoms were reversed, and a therapeutic effect on diabetic eye diseases was observed. At this time, that the SOST gene is knockdown may mean that sclerostin is reduced to a level below the level generally understood as an average level in the art. Accordingly, the activity inhibitor or expression inhibitor of the present invention does not mean only the complete inhibition of expression of the SOST gene or only the reduction of the level of sclerostin. In the present invention, the sclerostin inhibitor or SOST expression inhibitor may comprise all substances that reduce the amount/activity level of sclerostin to a level below that generally understood in the art.

The characteristic of the present invention is based on the fact that, when the expression of sclerostin is inhibited, the symptoms of diabetic eye diseases that appear under high glucose conditions or VEGF treatment conditions are treated, and is not in the specific type of the sclerostin inhibitor. Therefore, it is apparent that a person of ordinary skill in the art would understand that the same diabetic eye disease therapeutic activity as the present invention would be exhibited according to the application of general techniques capable of inhibiting the expression or activity of sclerostin in the art.

In one embodiment of the present invention, wherein the substance may be at least one selected from the group consisting of small interfering RNA (siRNA), short hairpin RNA (shRNA), micro RNA (miRNA), antisense oligonucleotides (ASO), Clustered Regularly Interspaced Short Palindromic Repeats (CRISPR/Cas), natural products, proteins, peptidomimetics, antibodies, exosomes, and compounds, but is not limited thereto. In the present invention, the "polynucleotide" or "nucleic acid" refers to deoxyribonucleic acid (DNA) or ribonucleic acid (RNA) in the form of a single strand or a double strand. Unless otherwise restricted, known analogues of natural nucleotides, which hybridize to nucleic acids in a manner similar to naturally occurring nucleotides, are also comprised.

In the present invention, the "siRNA" refers to an RNA molecule, generally having a length of 17-24 bp, which can interfere with the expression of a gene containing a nucleic acid sequence complementary to the sequence of the siRNA and inhibit expression in a process called RNA interference, but is not limited thereto.

In the present invention, siRNA may comprise both single-stranded siRNA and double-stranded siRNA. At this time, the double-stranded siRNA is derived from a longer double-stranded RNA molecule (dsRNA), and the dsRNA is cleaved by an endoribonuclease (Dicer) to form double-stranded siRNA, and a single-stranded siRNA may be formed in the RNA-induced silencing complex (RISC), but is not limited thereto.

In the present invention, methods for synthesizing or isolating and purifying siRNA, and methods for delivering siRNA, may be applied using methods generally used in the art, and, for example, may be various plasmids or non-viral vectors such as liposomes that are capable of being expressed in eukaryotic cells, but are not limited thereto.

In the present invention, the "shRNA" refers to a short hairpin RNA having a loop structure that induces an RNAi phenomenon and is a substance that induces degradation of mRNA by binding complementarily to a target mRNA. The shRNA may be microRNA-adapted shRNAs or simple hairpin shRNAs, but is not limited thereto, and comprises all modified forms to improve the function of shRNA.

In the present invention, the "miRNA" refers to a single-stranded RNA molecule generally having a length of approximately 22 nucleotides (nt), which forms a complex with Argonaute protein (AGO) and binds to the 3'UTR of target mRNA to suppress the expression of the corresponding gene.

In the present invention, the "antisense oligonucleotide" refers to a drug having a potential ability to realize personalized medicine together with RNAi therapeutics, and is a single-stranded nucleic acid polymer synthesized with an average of 12 to 25 nucleotides, which specifically has sequence complementarity to the target RNA and can regulate protein expression through various mechanisms. Also, the antisense oligonucleotide, which is a single-stranded ASO, may regulate gene expression depending on the chemical or binding site and the target, or may affect mRNA splicing through various mechanisms. When the ASO complementarily binds to the pre-mRNA target region, ribonuclease H (RNase H), which hydrolyzes the RNA strand of the RNA-DNA heteroduplex, is activated to degrade the target mRNA and suppress protein expression, but is not limited thereto, and the ASO may directly inhibit the target region or may interfere with the interaction with RNA-binding proteins by inducing steric hindrance through the binding of the ASO to mRNA. In the present invention, the antisense oligonucleotide may be modified for use as phosphorothioate (PS), PMO (Phosphorodiamidate morpholino oligonucleotide), PNA (Peptide nucleic acid), locked nucleic acid (LNA), or ribose modifications, but is not limited thereto, and comprises all modifications to which techniques generally applicable in the art to improve functionality are applied.

In the present invention, the "CRISPR/Cas" may refer to a 'guide RNA (gRNA)' made of RNA and 'Cas', which is an enzyme that cleaves DNA, and, for example, may be Cas9, Cas11, or Cas12, but is not limited thereto, and Cas may comprise, in a broad sense, all forms of enzymes that can be used in the CRISPR system.

In the present invention, the term "Cas9" refers to "CRISPR-Cas9", and CRISPR-Cas9, as a third-generation gene-editing tool, recognizes and cleaves and edits a specific nucleotide sequence to be used, and can be usefully used to simply, rapidly, and efficiently perform operations of inserting a specific gene into a target site of a genome or stopping the activity of a specific gene.

The Cas9 protein or gene information may be obtained from publicly known databases such as GenBank of the National Center for Biotechnology Information (NCBI), but is not limited thereto. In addition, the Cas9 protein can be appropriately modified by those skilled in the art to link additional domains as needed for specific purposes.

In the present invention, the Cas9 protein may comprise not only wild-type Cas9 but also all variants of Cas9 having the function of a nuclease for gene editing, and the origin of the Cas9 protein is not limited, and, as a non-limiting example, may be derived from *Streptococcus pyogenes, Francisella novicida, Streptococcus thermophilus, Legionella pneumophila, Listeria innocua, or Streptococcus mutans,* and may be appropriately selected and used by a person skilled in the art, but is not limited thereto.

In the present invention, the "gRNA" refers to a single-stranded RNA that locates a specific position of a target DNA to be edited and guides the Cas protein to the target DNA, and the gRNA may be adjacent to a PAM (protospacer adjacent motif) site and may comprise a sequence complementary to the 10-25 bp nucleotide sequence of the DNA to be edited. The guide RNA may have 1 to 3 additional nucleotides, more specifically 2 or 3 nucleotides, in front of the 5' region of the sequence capable of forming base pairs with the complementary strand of the target DNA sequence. In addition, the guide RNA may comprise a portion having a sequence complementary to the sequence in the target DNA (also referred to as a spacer region, target DNA recognition sequence, or base pairing region) and a hairpin structure for binding to the Cas protein. More specifically, it may comprise a portion having a sequence complementary to the sequence in the target DNA, a hairpin structure for Cas protein binding, and a terminator sequence, but is not limited thereto.

The gRNA of the present invention may target the sclerostin gene, and may be configured as a pair to simultaneously target different regions of the gene, but is not limited thereto.

In the present invention, the "natural product" may mean a product derived from a living organism such as animals and plants that live on land or in the ocean, or a product originating from a living organism such as cells or tissue culture products, and may mean something in its natural state without artificial processing, and the origin is not particularly limited as long as it is an extract of a natural substance, and, for example, may be a plant extract, an animal extract, a microbial extract, or a mineral extract. In addition, the natural substance may mean the substance itself, but is not limited thereto, and may mean all modified forms including the pharmacologically active ingredient of the natural substance. For example, it may be an extract, or a fraction thereof, a fermentation product, or a juice, but is not limited thereto.

In the present invention, the conditions such as the solvent of the extract are not particularly limited, and all conditions that can be generally used by a person skilled in the art may be applied according to the characteristics of the natural product or to allow inclusion of the pharmacologically active ingredient.

In the present invention, the method for fractionating into a fraction is not particularly limited as long as it is a method for fractionating an extract that is commonly used, and, as a non-limiting example, may be fractionated using one or more methods selected from the group consisting of chromatography separation methods, fraction separation methods using solubility differences, separation methods using specific gravity differences, centrifugation methods using density differences, solid sample extraction methods, and separation methods using color differences.

In the present invention, the "protein" is used compatibly with "polypeptide" or "peptide", and, for example, refers to a polymer of amino acid residues as generally found in proteins in a natural state, and may comprise proteins produced by recombinant expression vectors, but is not limited thereto.

In the present invention, the "recombinant expression vector" refers to a bacterial plasmid, phage, yeast plasmid, plant cell virus, mammalian cell virus, or other vectors. In general, any plasmid and vector may be used as long as it can be replicated and stabilized in a host. The recombinant expression vector of the present invention may preferably comprise a promoter, which is a transcription initiation factor to which RNA polymerase binds, any operator sequence for regulating transcription, a sequence encoding a suitable mRNA ribosome binding site, a sequence regulating the termination of transcription and translation, and a terminator, and the expression vector including the recombinant expression vector and appropriate transcription/translation regulatory signals may be constructed by methods known to those skilled in the art.

In addition, genes for tags to increase the production of recombinant proteins, genes for tags to maintain the structural stability of recombinant proteins, genes for tags to facilitate the separation of recombinant proteins, and selection marker genes such as antibiotic resistance genes for selecting transformants may be additionally comprised, and the tags for facilitating separation may comprise, but are not limited to, Avi tag, Calmodulin tag, polyglutamate tag, E tag, FLAG tag, HA tag, His tag, Myc tag, Stag, SBP tag, IgG-Fc tag, CTB tag, Softag 1 tag, Softag 3 tag, Strep tag, TC tag, V5 tag, VSV tag, and Xpress tag. The selection marker genes may comprise, as representative examples, herbicide resistance genes such as glyphosate or phosphinothricin, antibiotic resistance genes such as kanamycin, G418, Bleomycin, hygromycin, or chloramphenicol, and the aadA gene. The promoters may comprise, as representative examples, the pEMU promoter, MAS promoter, histone promoter, CIp promoter, cauliflower mosaic virus-derived 35S promoter, cauliflower mosaic virus-derived 19S RNA promoter, plant actin protein promoter, ubiquitin protein promoter, Cytomegalovirus (CMV) promoter, Simian virus 40 (SV 40) promoter, Respiratory syncytial virus (RSV) promoter, and Elongation factor-1 alpha (EF-1α) promoter. The terminators may comprise, as representative examples, the nopaline synthase (NOS) terminator, rice amylase RAmy1 A terminator, phaseolin terminator, terminator of the octopine gene of *Agrobacterium tumefaciens,* and rrnB1/B2 terminator of *Escherichia coli,* but the types of additionally comprised genes are not limited as long as they are types used in the manufacture of recombinant proteins.

In the present invention, methods for transforming with a recombinant vector, and methods for producing a desired protein from a transformant transformed with a recombinant vector, may all be possible as long as they are methods known in the art, and, at this time, depending on the characteristics of the recombinant vector, the transformant may be an animal or a plant, but is not limited thereto, and any method may be applied as long as it is for obtaining a functionally excellent protein at a high yield from the transformant.

In the present invention, the "peptidomimetic" means a substance designed to mimic a biologically active peptide, and may be a non-natural amino acid or other specific compound in order to stabilize the structure or alter the biological activity, but is not limited thereto. The peptidomimetic may be modified to enhance the target specificity and stability of the peptide and reduce degradability. For example, bending may be restricted to limit the conformational mobility of the peptide, or a non-natural compound may be comprised in the structure to reduce the possibility of degradation by enzymes, but is not limited thereto.

In the present invention, the "antibody" is a term compatible with "antibody fragment", and means an antibody that naturally exists in the body, but is not limited thereto, and comprises all antibodies obtained from human antibody phage libraries and human antibody-producing transgenic animals produced by advances in genetic engineering, cell engineering, and developmental engineering technologies. In addition, the antibody fragment refers to something that comprises a portion of an antibody and can perform the same function as the antibody, and, for example, may be Fab, Fab', F(ab')₂, scFv, diabody, dsFv, or a peptide comprising CDR, but is not limited thereto. The antibody fragment may be prepared by applying methods generally performed in the art.

In the present invention, the "exosome" may mean that it is discharged from somatic cells derived from human tissues including neurosphere, fibroblast, epithelial cell, muscle cell, cardiac cell, renal cell, neuronal cell, hair cell, hair root cell, hair follicle cell, epithelial cell, beta cell, gastric mucosal cell, goblet cell, G cell, immune cell, zymogenic cell, mast cell, endothelial cell, etc., but is not limited thereto. In addition, the exosome may mean that it is discharged from cells extracted from human body fluids such as urine, saliva, sweat, and blood, or that it is discharged from bone marrow-derived stem cells such as neural cord blood, adipose-derived stem cells, adult stem cells, embryonic stem cells, pluripotent stem cells such as iPSCs, or placental stem cells, but is not limited thereto. In addition, in order to facilitate the discharge of exosomes or increase the number of discharged exosomes, or to improve exosome secretion or functionality, physical stimulation such as electrical stimulation, heat, sound waves, laser, oxidative stress, LED light, chemical stimulation through treatment with chemical substances, or biological stimulation through treatment with substances such as enzymes acting on in vivo mechanisms, may be applied, but is not limited thereto. In the present invention, the size of the exosome may be a general exosome size, but is not limited thereto, and may vary depending on the discharge method, stimulation method, or culture method, and may vary depending on the amount or type of the loaded substance.

In the present invention, any method for stimulating, secreting, and obtaining exosomes may be applied as long as it is obvious to a person skilled in the art, and is not limited thereto.

In the present invention, the "compound" means a substance formed by the chemical bonding of two or more different kinds of elements, and comprises both organic compounds and inorganic compounds. The compound of the present invention may mean any chemical substance that can inhibit the expression or activity of sclerostin.

In the present invention, the "sclerostin inhibitor" may be delivered to target cells through a method or carrier that can be generally used in the art. For example, it may be delivered by a vector, but is not limited thereto. In addition, the substance of the present invention may be delivered through a method or carrier that can be generally applied according to the dose, cycle, age, gender, clinical symptoms, and disease progression state of the user, but is not limited thereto.

In one embodiment of the present invention, wherein the diabetic eye disease may be at least one selected from the group consisting of diabetic retinopathy, diabetic macular edema, glaucoma, and cataracts, but is not limited thereto.

In the present invention, the "diabetic retinopathy" is a retinal disease caused by diabetes and is classified into non-proliferative and proliferative types

In the present invention, the "non-proliferative diabetic retinopathy" refers to most diabetic retinopathy, and is characterized in that the retinal capillaries enlarge like small balloons and become deformed into a saccular shape. The progression stage of non-proliferative diabetic retinopathy is divided into three stages, and the retinal vascular occlusion tends to become more severe. In general, the non-proliferative type does not cause blindness, but if the capillary walls become weakened and water leaks out, the macula becomes swollen, resulting in visual impairment. In general, the non-proliferative type does not require treatment, but if macular edema occurs, treatment must be accompanied.

In addition, the "proliferative diabetic retinopathy" refers to a condition in which, in some diabetic patients, retinopathy may progress to a more severe form, proliferative diabetic retinopathy, after several years. In the proliferative type, as retinal blood vessels are occluded and damaged, neovascularization grows from the retina toward the vitreous. The neovascular vessels are fragile, so they easily rupture and blood leaks, obscuring vision, which is called vitreous hemorrhage. In addition, the neovascular vessels grow together with fibrous tissue, and when the fibrous tissue contracts and pulls, the retina may become wrinkled or the retina may detach, causing retinal detachment. Neovascularization may also occur in the iris and become a cause of severe glaucoma. Even when severe retinal damage has already progressed, changes in vision may not be felt. In the case of non-proliferative diabetic retinopathy, most patients do not feel symptoms, but even in the proliferative type, there are cases in which the patient is unaware until it is already too late for treatment.

Blood glucose control, blood pressure, the duration of diabetes, and constitutional characteristics are closely related to the occurrence of diabetic retinopathy. It is known that if diabetes has been suffered for a long time, the probability of developing retinopathy is high. Most patients with type 1 diabetes eventually develop non-proliferative diabetic retinopathy, and the same is true for type 2 diabetes.

In the present invention, the "diabetic macular edema (DME)" refers to a disease in which edema occurs in the macular region, which is the center of the retina, and acts as the biggest cause of visual impairment in patients with diabetic retinopathy. It occurs due to the increase in the permeability of retinal blood vessels and the occurrence of microaneurysms as a microvascular complication caused by diabetes, and blood plasma proteins and lipid components leak into the retinal tissue. As the above substances accumulate in the macular region, which is the center of the retina and plays the most important role in vision, the thickness of the retina increases and damages neural connections, thereby causing visual impairment.

In the present invention, the "glaucoma" or "cataract" may be diabetic glaucoma or diabetic cataract induced by diabetes. Accordingly, in the present invention, glaucoma or cataract may be diabetic glaucoma or diabetic cataract. Diabetic patients are known to have a more than 40% higher probability of developing glaucoma than the general population, and the probability further increases the longer diabetes is suffered and the older the patient becomes. In addition, cataract also occurs at a higher frequency, may occur at a younger age, and is characterized by rapid progression.

In one embodiment of the present invention, wherein the composition may be any one formulation selected from the group consisting of suspensions, gels, ointments, injectable solutions, sprays, and eye drops, but is not limited thereto.

In one embodiment of the present invention, wherein the composition may inhibit inflammatory responses and microvascular complications, but is not limited thereto.

The main objective of the present invention may be to suppress inflammatory responses and microvascular complications by reducing inflammatory cytokines or angiogenesis through inhibition of sclerostin under hyperglycemic conditions. The inflammatory cytokines may be those increased by diabetes. In one embodiment of the present invention, it was confirmed that inflammatory cytokines significantly increased by high glucose conditions or VEGF treatment, and the expression level of inflammatory cytokines decreased by inhibition of sclerostin. In addition, it was confirmed that angiogenesis was inhibited as vascular endothelial marker expression was suppressed and tube formation was inhibited by the reduction of angiogenic activity.

Hyperglycemia causes microvascular damage and induces the release of inflammatory cytokines, which is a characteristic of angiopathy. In the present invention, high glucose conditions were induced in HREC using a high concentration of glucose (30 mM), and changes in molecular expression were evaluated. Hyperglycemia can induce an inflammatory state in both type 1 and type 2 DM, and pancreatic beta cells may be degraded due to the elevated level of cytokines. The serum levels of inflammatory cytokines are significantly high in DM patients. In in vivo studies, high glucose exposure greatly enhanced the mRNA and protein expression of TNF-α, which further increased the expression of sclerostin in an NF-κB-dependent manner under hyperglycemic conditions.

In one embodiment of the present invention, wherein the composition may be characterized by one or more of the following, but is not limited thereto:
inhibition of inflammatory cytokines within retinal cells;
inhibition of angiogenesis;
inhibition of vascular endothelial growth factor and vascular adhesion markers; and
reduction of oxidative stress.

In the present invention, the term "sclerostin (Sclerostin, hereinafter used compatibly with SOST)" is a protein encoded by the SOST gene. Since it is a secreted glycoprotein preferentially produced by osteocytes, SOST is known to inhibit bone formation.

Dickkopf family proteins (DKK) and sclerostin bind to the co-receptor low-density lipoprotein receptor-related protein (LRP) 5/6 and are two additional Wnt antagonists that respectively inhibit Wnt signaling. Sclerostin, a well-known antagonist of Wnt signaling, promotes angiogenesis through LRP6. Furthermore, it increases VEGF and PIGF, which further promote angiogenesis. In one embodiment of the present invention, it was shown that silencing of sclerostin reduced retinal endothelial angiogenic activity and vascular formation ability. In addition, it was shown that treatment with SOST siRNA reduced tube formation, thereby reducing vascular formation. Furthermore, in the present invention, it was demonstrated that sclerostin reduces inflammation by minimally inhibiting Wnt/β-catenin.

The pharmaceutical composition according to the present invention may be formulated and used in the form of powders, granules, sustained-release granules, enteric granules, liquids, eye drops, elixirs, emulsions, suspensions, tinctures, troches, aromatic waters, lemonades, tablets, sustained-release tablets, enteric tablets, sublingual tablets, hard capsules, soft capsules, sustained-release capsules, enteric capsules, pills, tinctures, soft extracts, dry extracts, fluid extracts, injections, capsules, perfusion solutions, eye drops, lotions, pastes, sprays, inhalants, patches, sterile injectable solutions, or aerosols, respectively, according to conventional methods, and the external formulations may have the form of creams, gels, patches, sprays, ointments, eye drops, lotions, liniments, pastes, or cataplasms.

The carriers, excipients, and diluents that may be comprised in the pharmaceutical composition according to the present invention may comprise lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

When formulated, it is prepared using diluents or excipients such as fillers, extenders, binders, wetting agents, disintegrants, and surfactants that are commonly used.

As additives for tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, calcium hydrogen phosphate, calcium sulfate, sodium chloride, sodium bicarbonate, refined lanolin, microcrystalline cellulose, dextrin, sodium alginate, methyl cellulose, sodium carboxymethyl cellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel may be used; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethyl cellulose, sodium methyl cellulose, methyl cellulose, microcrystalline cellulose, dextrin, hydroxycellulose, hydroxypropyl starch, hydroxyethylcellulose, purified shellac, starch paste, hydroxypropyl cellulose, hydroxypropyl methylcellulose, polyvinyl alcohol, and polyvinylpyrrolidone may be used; disintegrants such as hydroxypropyl methylcellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, anhydrous silica, 1-hydroxypropyl cellulose, dextran, ion exchange resin, polyvinyl acetate, casein treated with formaldehyde, and gelatin, alginic acid, amylose, guar gum, baking soda, polyvinylpyrrolidone, calcium phosphate, gelatinized starch, gum arabic, amylopectin, pectin, sodium polyphosphate, ethyl cellulose, white sugar, magnesium aluminum silicate, D-sorbitol solution, and anhydrous colloidal silica may be used; and lubricants such as calcium stearate, magnesium stearate, stearic acid, hydrogenated vegetable oil, talc, lycopodium, kaolin, petrolatum, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, macrogol, synthetic aluminum silicate, anhydrous colloidal silica, higher fatty acids, higher alcohols, silicone oil, paraffin oil, polyethylene glycol fatty acid ether, starch, sodium chloride, sodium acetate, sodium oleate, dl-leucine, and anhydrous colloidal silica may be used.

As additives for liquid formulations according to the present invention, water, dilute hydrochloric acid, dilute sulfuric acid, sodium citrate, sucrose monostearate, polyoxyethylene sorbitol fatty acid esters (Tween esters), polyoxyethylene monoalkyl ethers, lanolin ethers, lanolin esters, acetic acid, hydrochloric acid, ammonia solution, ammonium carbonate, potassium hydroxide, sodium hydroxide, prolamine, polyvinylpyrrolidone, ethyl cellulose, and sodium carboxymethyl cellulose may be used.

In the syrup formulation according to the present invention, a solution of white sugar, other sugars, or sweeteners may be used, and, if necessary, flavoring agents, coloring agents, preservatives, stabilizers, suspending agents, emulsifying agents, and viscosity-increasing agents may be used.

In the emulsion formulation according to the present invention, purified water may be used, and, if necessary, emulsifying agents, preservatives, stabilizers, and flavoring agents may be used.

In the suspension formulation according to the present invention, suspending agents such as acacia, tragacanth, methyl cellulose, carboxymethyl cellulose, sodium carboxymethyl cellulose, microcrystalline cellulose, sodium alginate, hydroxypropyl methylcellulose (HPMC), HPMC 1828, HPMC 2906, and HPMC 2910 may be used, and, if necessary, surfactants, preservatives, stabilizers, coloring agents, and flavoring agents may be used.

In the injectable formulation according to the present invention, solvents such as distilled water for injection, 0.9% sodium chloride injection, Ringer's injection, dextrose injection, dextrose + sodium chloride injection, PEG (polyethylene glycol), lactated Ringer's injection, ethanol, propylene glycol, non-volatile oils such as sesame oil, cottonseed oil, peanut oil, soybean oil, corn oil, ethyl oleate, isopropyl myristate, and benzyl benzoate may be used;solubilizing aids such as sodium benzoate, sodium salicylate, sodium acetate, urea, urethane, monoethylacetamide, butazolidin, propylene glycol, Tween types, nicotinic acid amide, hexamine, and dimethylacetamide may be used; buffers such as weak acids and their salts (acetic acid and sodium acetate), weak bases and their salts (ammonia and ammonium acetate), organic compounds, proteins, albumin, peptone, and gums may be used; isotonic agents such as sodium chloride may be used; stabilizers such as sodium bisulfite (NaHSO₃), carbon dioxide gas, sodium metabisulfite (Na₂S₂O₅), sodium sulfite (Na₂SO₃), nitrogen gas (N₂), and ethylenediaminetetraacetic acid may be used; sulfurizing agents such as sodium bisulfide 0.1%, sodium formaldehyde sulfoxylate, thiourea, ethylenediaminetetraacetic acid disodium, and acetone sodium bisulfite may be used; anesthetics such as benzyl alcohol, chlorobutanol, procaine hydrochloride, glucose, and calcium gluconate may be used; and suspending agents such as sodium CMC (carboxymethyl cellulose), sodium alginate, Tween 80, and aluminum monostearate may be comprised.

In the suppository formulation according to the present invention, bases such as cocoa butter, lanolin, Witepsol, polyethylene glycol, glycerogelatin, methyl cellulose, carboxymethyl cellulose, a mixture of stearic acid and oleic acid, Subanal, cottonseed oil, peanut oil, palm oil, cacao butter + cholesterol, lecithin, lanette wax, monoglyceride stearate, Tween or Span, Imhausen, Monolen (propylene glycol monostearate), glycerin, Adeps solidus, Butyrum Tego-G, Cebes Pharma 16, Hexaryde Base 95, Cotomar, Hydrokote SP, S-70-XXA, S-70-XX75 (S-70-XX95), Hydrokote 25, Hydrokote 711, Idropostal, Massa estrarium (A, AS, B, C, D, E, I, T), Massa-MF, Masupol, Masupol-15, Neosuppostal-N, Paramount-B, Supposiro (OSI, OSIX, A, B, C, D, H, L), Suppository base IV type (AB, B, A, BC, BBG, E, BGF, C, D, 299), Suppostal (N, Es), Wecobee (W, R, S, M, Fs), and triglyceride bases such as Tegester TG-95, MA, and 57 may be used.

Solid formulations for oral administration comprise tablets, pills, powders, granules, and capsules, and such solid formulations are prepared by mixing the above extract with at least one excipient, for example, starch, calcium carbonate, sucrose, lactose, or gelatin. In addition to simple excipients, lubricants such as magnesium stearate and talc are also used.

Liquid formulations for oral administration comprise suspensions, internal solutions, emulsions, and syrups, and may comprise, in addition to commonly used simple diluents such as water and liquid paraffin, various excipients such as wetting agents, sweeteners, flavoring agents, and preservatives. Formulations for parenteral administration comprise sterile aqueous solutions, non-aqueous solvents, suspensions, emulsions, freeze-dried preparations, and suppositories. Non-aqueous solvents and suspensions may comprise propylene glycol, polyethylene glycol, vegetable oils such as olive oil, and injectable esters such as ethyl oleate.

The pharmaceutical composition according to the present invention is administered in a pharmaceutically effective amount. In the present invention, the "pharmaceutically effective amount" means an amount sufficient to treat a disease at a reasonable benefit/risk ratio applicable to medical treatment. Accordingly, in the present invention, the "pharmaceutically effective amount" may be used compatibly with the term "therapeutically effective amount." The effective dosage level may be determined depending on factors including the type and severity of the patient's disease, the activity of the drug, the sensitivity to the drug, the time of administration, the route of administration and excretion rate, the treatment period, the medication used in conjunction, and other factors well known in the medical field.

The pharmaceutical composition according to the present invention may be administered as an individual therapeutic agent or in combination with other therapeutic agents, and may be administered sequentially or simultaneously with conventional therapeutic agents, and may be administered as a single or multiple dose. It is important to administer an amount that can obtain the maximum effect with a minimum amount without side effects by considering all of the above factors, and this can be easily determined by a person skilled in the art to which the present invention pertains.

The pharmaceutical composition of the present invention may be administered to a subject through various routes. All modes of administration may be envisaged, for example, oral administration, subcutaneous injection, intraperitoneal administration, intravenous injection, intramuscular injection, intracerebroventricular (epidural) injection, sublingual administration, buccal administration, rectal insertion, vaginal insertion, eye administration, auricular administration, nasal administration, inhalation, spray through the mouth or nose, dermal administration, and transdermal administration.

The pharmaceutical composition of the present invention is determined according to the type of drug as the active ingredient, together with various relevant factors such as the disease to be treated, the route of administration, the age, gender, weight, and severity of the disease of the patient.

In the present invention, the "subject" means a target requiring treatment of a disease, and, more specifically, means a mammal such as a human or a non-human primate, mouse, rat, dog, cat, horse, or cow.

In the present invention, the "administration" means providing a predetermined composition of the present invention to a subject by any appropriate method.

In the present invention, the "prevention" means all acts of suppressing or delaying the onset of a desired disease, and the "treatment" means all acts in which the desired disease and the associated metabolic abnormalities are improved or beneficially changed by the administration of the pharmaceutical composition according to the present invention, and the "improvement" means all acts of reducing parameters related to the desired disease, for example, the degree of symptoms, by the administration of the composition according to the present invention.

The present invention provides a food composition comprising a sclerostin inhibitor as an active ingredient for prevention or improvement of diabetic eye disease.

In one embodiment of the present invention, wherein the food may be a health functional food, but is not limited thereto.

In one embodiment of the present invention, wherein the substance may be at least one selected from the group consisting of small interfering RNA (siRNA), short hairpin RNA (shRNA), micro RNA (miRNA), antisense oligonucleotides (ASO), Clustered Regularly Interspaced Short Palindromic Repeats (CRISPR/Cas), natural products, proteins, peptidomimetics, antibodies, exosomes, and compounds, but is not limited thereto.

In one embodiment of the present invention, wherein the diabetic eye disease may be at least one selected from the group consisting of diabetic retinopathy, diabetic macular edema, glaucoma, and cataract, but is not limited thereto.

The health food and health functional food composition comprising the active substance according to the present invention may be added directly to food or used together with other food or food ingredients, and may be appropriately used according to general methods. In addition, the mixing amount of the active substance may be appropriately determined according to the intended use (for example, for prevention or improvement).

Generally, the amount of the composition in the health food or health functional food may be added in an amount of 0.1 to 90 parts by weight based on 100 parts by weight of the total food weight. However, in the case of long-term intake for the purpose of maintaining or regulating health, the amount may be less than the above range, and in terms of safety, the active ingredient according to the present invention may be used in an amount more than the above range.

In the health food and health functional food composition of the present invention, except for containing the active substance of the present invention as an essential component in the indicated ratio, other components are not particularly limited, and various flavoring agents or natural carbohydrates, as in ordinary beverages, may be comprised as additional components. Examples of the natural carbohydrates comprise conventional sugars such as monosaccharides, for example, glucose, fructose, etc.; disaccharides, for example, maltose, sucrose, etc.; and polysaccharides, for example, dextrin, cyclodextrin, etc.; and sugar alcohols such as xylitol, sorbitol, and erythritol. As the flavoring agents other than those described above, natural flavoring agents (thaumatin, stevia extract (for example, rebaudioside A, glycyrrhizin, etc.)) and synthetic flavoring agents (saccharin, aspartame, etc.) may be used. The ratio of the natural carbohydrates may generally be about 1 to 20 parts by weight, preferably about 5 to 12 parts by weight, per 100 parts by weight of the health functional food composition of the present invention, but is not limited thereto.

In addition to the above, the health food and health functional food composition comprising the active substance of the present invention may further comprise various nutrients, vitamins, minerals (electrolytes), synthetic and natural flavoring agents, coloring agents and bulking agents (cheese, chocolate, etc.), pectic acid and salts thereof, alginic acid and salts thereof, organic acids, protective colloidal thickeners, pH regulators, stabilizers, preservatives, glycerin, alcohol, and carbonating agents used in carbonated beverages. In addition, the health food and health functional food composition of the present invention may comprise fruit pulp for the manufacture of natural fruit juices, fruit juice beverages, and vegetable beverages.

Such components may be used independently or in combination, and are generally selected in the range of 0.1 to about 20 parts by weight per 100 parts by weight of the health food or health functional food composition containing the active substance of the present invention, but are not limited thereto.

The present invention provides a kit comprising a sclerostin inhibitor and instructions for prevention or treatment of diabetic eye disease.

The "kit" of the present invention may, in addition to the above components, further comprise other components, devices, or substances that are conventionally necessary for storage, management, or enhancement of the efficacy of the composition of the present invention. In addition, all components comprised in the kit may be used without limitation in the number of times of use, there is no limitation in the order of use of each substance, and the application of each substance may be performed simultaneously or may be performed sequentially.

The kit of the present invention may comprise a container in addition to the formulation and the instruction manual. The container may serve to package the components, and may also serve to store and secure them. The material of the container may take the form of, for example, a bottle, tub, sachet, envelope, tube, or ampoule, and these may be formed partially or entirely from plastic, glass, paper, foil, or wax. The container may be equipped with a cap that is fully or partially detachable, initially being part of the container or attached to the container by mechanical, adhesive, or other means, and may also be equipped with a stopper that allows access to the contents by means of a syringe needle. The kit may comprise an outer package, and the outer package may comprise instructions concerning the use of the components, but is not limited thereto.

The terms used in the present invention have been selected as generally used terms that are currently widely used, while considering their functions in the present invention; however, they may vary depending on the intention of a person skilled in the art, precedents, or the emergence of new technologies. In addition, in certain cases, terms arbitrarily selected by the applicant are used, and in such cases, the meanings thereof will be described in detail in the description of the invention. Accordingly, the terms used in the present invention should be defined based on the meanings of the terms and the overall contents of the present invention, rather than merely by their names.

Throughout the specification of the present invention, when a part is described as "comprising" a certain component, it means that, unless specifically stated otherwise, other components are not excluded but may be further comprised. The terms of degree, such as "about" and "substantially," used throughout the specification of the present invention, are used in the sense of being at or close to the numerical value when manufacturing and material tolerances inherent in the stated meaning are presented, and are used to prevent an unscrupulous infringer from unfairly utilizing the disclosed content in which accurate or absolute numerical values are mentioned for the purpose of assisting understanding of the present invention.

Throughout the specification of the present invention, the term "combinations thereof" comprised in Markush-type expressions means a mixture or combination of at least one selected from the group consisting of the components described in the Markush-type expression, and means that one or more selected from the group consisting of the components are comprised.

### MODE FOR CARRYING OUT INVENTION

The following describes a preferred embodiment to aid in understanding the present invention. However, the embodiments described below are provided solely to facilitate a clearer understanding of the invention and do not limit the scope of the invention.

### Examples

### Antibodies and Chemical Reagents

The list of antibodies and chemical reagents used in the present invention is shown in Table 1 below.

**Table 1**

| **Product name** | **Company name** |
|---|---|
| Fetal bovine serum | Welgene(Gyeongsan-si, Gyeongsangbukdo, South Korea) |
| Dulbecco's phosphate-buffered saline | |
| 1X Trypsin-EDTA solution | |
| Dimethyl sulfoxide(DMSO) | Sigma-Aldrich(St. Louis, MO, USA) |
| 3-[4, 5-dimethyl-2-thiazolyl]-2,5-diphenyltetrazoliumbromide(MTT) | |
| Paraformaldehyde(4%) | Biosesang(Seongnam-si, Gyeonggi-do, South Korea) |
| Matrigel | BD Biosciences(Bedford, MA, USA) |
| Human retinal endothelial primary cells(HREC, 36205-33) | Celprogen Inc,(Torrance, CA, USA). |
| Human retinal endothelial cell culture complete media with serum and antibiotic | |
| Human retinal endothelial cell culture | |
| complete media without serum and antibiotic | |
| Antibody against β-Catenin | Cell Signaling Technology(Danvers, Massachusetts, USA) |
| Halt^{™} Protease and Phosphatase Inhibitor Cocktail | Thermo Fisher Scientific(Rockford, IL, USA) |
| Pierce^{™} RIPA Buffer | |
| Pierce^{™} BCA Protein Assay Kit | |
| Opti-MEM^{™} reduced serum medium | Thermo Fisher Scientific(Grand Island, NY, USA). |
| Phenylmethylsulphonyl fluoride | Roche(Mannheim, Germany) |
| Human VEGF₁₆₅ | (PeproTech, Cranbury, NJ, USA) |
| Mounting Medium with 4',6-diamidino-2-phenylindole(DAPI)-Aqueous Fluoroshield | Abcam(Cambridge, MA, USA) |
| Antibodies against VEGFA, LRP6, Wnt3a, and COX-2 | |
| Antibodies against TNF-α and Wnt5a | R&D Systems(Minneapolis, MN, USA) |
| Sclerostin siRNA(h) | Santa Cruz Biotechnology(Dallas, TX, USA). |
| Sclerostin-CRISPR activation plasmid(h) | |
| UltraCruz Transfection Reagent | |
| Plasmid Transfection Medium | |
| Antibodies against SOST, ICAM-1, IL-6, and β-actin | |
| Mouse anti-goat IgG-HRP | |
| Mouse anti-rabbit IgG-HRP | |
| Goat anti-mouse IgG- conjugated with HRP | |
| Lipofectamine RNAiMAX reagent | Invitrogen(Carlsbad, CA, USA) |
| Alexa Fluor 594 donkey anti-goat IgG(H&L) | |
| Alexa Fluor 594 goat anti-mouse IgG(H&L) | |
| Antibodies against MCP-1, NFκB p65, and iNOS | |
| TB Green^{®} Premix Ex Taq^{™} | Takara Bio Inc.,(Kusatsu, Shiga, Japan) |
| The protein loading buffer | Bio-Rad Laboratories, Inc.(Hercules, CA, USA) |
| iScript cDNA synthesis kit | |
| The RNA extraction kit Ribospin II | GeneAll Biotechnology(South Korea) |

### Cell culture

Human retinal endothelial cells (HREC) were grown in human retinal endothelial cell complete growth medium containing serum and antibiotics (Celprogen Inc., Torrance, CA, USA). The mononuclear cells were quantified using the trypan blue exclusion technique prior to seeding into extracellular matrix-coated T75 culture flasks (Celprogen Inc., Torrance, CA, USA) at a specific density of 1.0 × 10^6 mL⁻¹. In subsequent experiments, the cell seeding density was maintained the same. HREC were maintained in an incubator set at 37°C and 5% CO₂.

### Treatment of HRECs with high glucose level and VEGF

HRECs were plated in extracellular matrix-coated 6-well tissue culture plates and treated with human retinal endothelial cell complete growth medium containing serum and antibiotics (Celprogen Inc.) and maintained at 37°C in a humidified environment. A glucose concentration of 30 mM was prepared in cell growth medium and applied to 80% confluent cells for high glucose treatment and maintained for 24 hours. For VEGF treatment of HRECs, 50 ng of Human VEGF165 (PeproTech, Cranbury, NJ, USA) was prepared with cell growth medium and used after 80% cell confluency and maintained for 24 hours.

### Small Interfering RNA(siRNA) transfection

Human retinal endothelial cells (HRECs) were seeded in extracellular matrix-coated 6-well tissue culture plates using human retinal endothelial cell complete growth medium without serum and antibiotics (Celprogen Inc, Torrance, CA, USA). When the HRECs reached 50-60% confluence, according to the manufacturer's instructions, the cells were transfected using Lipofectamine RNAiMAX transfection reagent with Sclerostin siRNA (h) (sc-61503, a pool of three target-specific 19-25 nt siRNAs from Santa Cruz Biotechnology) and control siRNA at final concentrations of 10 nM, 30 nM, and 50 nM, according to the manufacturer's instructions. After transfection for 24 hours, HRECs were separately treated with 30 mM glucose and 50 ng VEGF for an additional 24 hours, followed by further experiments. Control siRNA used was sc-37007 (Santa Cruz, Dallas, Tx, USA).

### Cell viability assay

An MTT assay was performed using the standard test protocol [ISO10993-5:2009I] to evaluate in vitro cell viability after high glucose (HG) and siRNA transfection. After treatment with glucose and siRNA transfection for 24 hours, the MTT test was conducted. A previous protocol was used to determine cell viability (measured as optical density) (Sayed, S., et al., Thermal cycling effect on osteogenic differentiation of MC3T3-E1 cells loaded on 3D-porous Biphasic Calcium Phosphate (BCP) scaffolds for early osteogenesis. Mater Sci Eng C Mater Biol Appl, 2019. 105: p. 110027.). To generate formazan crystals, MTT solution (5 mg/mL in PBS) was added to the cell culture medium at a ratio of 1:9, and the mixture was incubated at 37°C for 4 hours. To extract formazan crystals, DMSO was added and incubated for 1 hour to dissolve. Finally, the absorbance was measured at 595 nm using an ELISA reader (EL,312, Biodynamics Reader; Bio-Tek Instruments, Winooski, VT, USA). A triple test was performed for each sample.

### Clustered regularly interspaced short palindromic repeats(CRISPR)-SOST knockin)

For sclerostin CRISPR activation, HRECs were plated into a 6-well tissue culture plate with extracellular matrix coating in human retinal endothelial cell complete culture medium without serum and antibiotics. After 50-60% confluency of HRECs, sclerostin-CRISPR activation plasmids (h) of the SAM transcription activation system (three target-specific 19-25 nt pools) from Santa Cruz Biotechnology and control CRISPR were used at the final concentration. According to the manufacturer's instructions, 10 ng, 30 ng, 50 ng, and 100 ng were introduced using UltraCruz Transfection Reagent and Plasmid Transfection Medium. After 24 hours, further testing was conducted.

### Quantitative RT-PCR

After cell treatment, total RNA was extracted using the Ribospin II RNA extraction kit (GeneAll Biotechnology). After measuring the RNA concentration using a NanoDrop-One spectrophotometer (Thermo Fisher Scientific, Cambridge, MA, USA), RNA was reverse-transcribed into cDNA using the iScript cDNA synthesis kit (Bio-Rad Laboratories, Inc., Hercules, CA, USA) in a SimpliAmp Thermal Cycler (Applied Biosystems, Singapore). All cDNA samples were stored at -20°C for subsequent gene expression studies. qRT-PCR was performed using TB Green^{®} Premix Ex TaqTM (Takara Bio Inc., Kusatsu, Shiga, Japan) and the CFX96TM RT-PCR detection system (Bio-Rad, Singapore). Real-time PCR tests were conducted in triplicate. After standardizing data to GAPDH levels, relative mRNA expression levels were estimated using the 2-ΔΔCt method. The qRT-PCR primer sequences used in the present invention are shown in Table 2 below.

**Table 2**

| **Seq.** | **Name** | **Sequence 5'-3'** |
|---|---|---|
| Seq. 1 | SOST_F | F- GAGCCTGTGCTACTGGAAGG |
| Seq. 2 | SOST_R | R- TGATTTCCGTGGCATCATT |
| Seq. 3 | TNF-α_F | F-GGTGCCCGCTGCTGTCTAAT |
| Seq. 4 | TNF-α_R | R- TGCAACGCGAGTCTGTGTTT |
| Seq. 5 | TNF-α_F | F-AAGCTGAGGGGCAGCTCCAGT |
| Seq. 6 | TNF-α_R | R- TCTGGTAGGAGACGGCGATGC |
| Seq. 7 | ICAM-1_F | F- CTTCGTGTCCTGTATGGCCC |
| Seq. 8 | ICAM-1_R | R- CACATTGGAGTCTGCTGGGA |
| Seq. 9 | VCAM-1_F | F- GTCAATGTTGCCCCCAGAGATA |
| Seq. 10 | VCAM-1_R | R- ACAGGATTTTCGGAGCAGGA |
| Seq. 11 | iNOS_F | F- GCTCTACACCTCCAATGTGACC |
| Seq. 12 | iNOS_R | R- CTGCCGAGATTTGAGCCTCATG |
| Seq. 13 | NFκB_F | F- CACTTATGGACAACTATGAGGTCTCTGG |
| Seq. 14 | NFκB_R | R- CTGTCTTGTGGACAACGCAGTGGAATTTTAGG |
| Seq. 15 | Cox-2_F | F- TTGCTGGCAGGGTTGCTGGTGGTA |
| Seq. 16 | Cox-2_R | R- CATCTGCCTGCTCTGGTCAATGGAA |
| Seq. 17 | IL-1β_F | F-TTGCTCAAGTGTCTGAAGCAGC |
| Seq. 18 | IL-1β_R | R- CTTGCTGTAGTGGTGGTCGG |
| Seq. 19 | IL-6_F | F- GGATTCAATGAGGAGACTTGCC |
| Seq. 20 | IL-6_R | R- GGGTCAGGGGTGGTTATTGC |
| Seq. 21 | MCP-1_F | F- GGCTGAGACTAACCCAGAAAC |
| Seq. 22 | MCP-1_R | R- GAATGAAGGTGGCTGCTATGA |
| Seq. 23 | LRP5_F | F-GTGACAATGGTGGCTGCTC |
| Seq. 24 | LRP5_R | R-TCCACAGGTCAGCAGGTTCT |
| Seq. 25 | LRP6_F | F-TCAATGATCAGCTCCCTCAG |
| Seq. 26 | LRP6_R | R-TAAGTGCCTTTGGTGCTTGA |
| Seq. 27 | CTNNB1_F | F-TCTCCCATTGAAAACATCCA |
| Seq. 28 | CTNNB1_R | R-CTGTGGCTCCCTCAGCTTC |
| Seq. 29 | Wnt3a_F | F-TGTTGGGCCACAGTATTCCT |
| Seq. 30 | Wnt3a_R | R-CACTCCTGGATGCCAATCTT |
| Seq. 31 | Wnt5a_F | F-TTAAGCCCAGGAGTTGCTTT |
| Seq. 32 | Wnt5a_R | R-CAATTACAACCTGGGCGAAG |
| Seq. 33 | GAPDH_F | F- GAGAAACCTGCCAAGTATGATGAC |
| Seq. 34 | GAPDH_R | R- AGAGTGGGAGTTGCTGTTGAAG |

### Western blot(WB) analysis

For WB analysis, total proteins were obtained using RIPA lysis buffer containing 1X protease and phosphatase inhibitor cocktail. The total protein concentration was determined using the BCA protein assay kit (Thermo Scientific, Rockford, Illinois, USA). After separating the protein samples using 8% and 12% SDS-PAGE, they were transferred onto a polyvinylidene difluoride (PVDF) membrane. Then, the membrane was treated overnight with specific primary antibodies, followed by incubation with specific secondary antibodies (HRP-conjugated). Bands were identified using chemiluminescence ECL reagents, and densitometric analysis was performed using the ChemiDocTM XRS+ system (Bio-Rad, Hercules, CA, USA). Anti-β-actin antibody served as a loading control.

### Immunocytochemical analysis

After cell treatment, HRECs transfected with SOST siRNA were fixed in 4% paraformaldehyde solution. The cells were permeabilized using 0.25% Triton X-100, and then the cells were blocked with 2.5% bovine serum albumin (BSA). Thereafter, according to the previous protocol, the cells were immunostained overnight at 4 °C with an antibody against mouse sclerostin (1:50). After incubation with the primary antibody, the cells were incubated with the secondary antibody (Alexa Fluor 594, 1:1000). After washing, the cells were mounted with Fluoroshield-DAPI aqueous solution. Images were observed using a fluorescence microscope (Leica DMi8) and Leica LAS AF software.

### Tube formation assay

Matrigel (phenol red-free) was coated onto a pre-cooled 48-well plate. The coating process was completed on ice. Then, it was stored in a 37°C incubator for 30 minutes. Pre-treated HRECs were collected and diluted in serum- and antibiotic-free medium (4×10⁵ cells/mL). After collection, the treated HRECs were seeded in triplicate onto the Matrigel-coated 48-well plate and incubated at 37°C. Tube formation of HRECs appeared after 8 hours. Phase-contrast images of tube formation were captured using a Leica DMi8 microscope.

### Statistical analyses

All studies were performed at least three times, and all data were expressed as mean ± SD (standard deviation). Significant differences were determined using Bonferroni's multiple comparison test following one-way analysis of variance (ANOVA) between the control group and other groups. Statistically significant differences were confirmed through multiple comparisons and indicated as follows: *P < 0.05, **P < 0.01, ***P < 0.001 versus control group, and #P < 0.05, ##P < 0.01, ###P < 0.001 for HG-treated or VEGF-treated samples, respectively. All statistical evaluations were performed using GraphPad Prism 8.

### Example 1. Effect of SOST knockdown on HRECs: Reduction of SOST expression and non-toxicity

The effect of SOST knockdown on human retinal endothelial cells (hereinafter, HRECs) was confirmed under high glucose (HG) conditions. For high glucose conditions, HRECs were treated with 30 mM glucose and 50 ng VEGF for 24 hours. The treatment of 30 mM glucose to induce high glucose conditions was selected from previous experiments (Int J Mol Sci. 2023 Mar 12;24(6):5428. doi: 10.3390/ijms24065428.). According to the study experiments, after 24 hours of 30 mM glucose treatment, more than 80% of cells survived, whereas less than 80% of cells survived with 60 mM glucose treatment. Treatment with 30 mM glucose for 24 hours increased TNF-α, NF-κB, MCP-1, IL-1β, IL-6, COX-2, iNOS, VEGFA, and adhesion markers VCAM-1 and ICAM-1. After treating HRECs with various concentrations of siRNA (10 nM, 30 nM, and 50 nM), the SOST knockdown effect was evaluated using qRT-PCR, WB, and immunostaining.

As a result, when treated with control siRNA (siCON+HG), the expression of SOST was shown to be higher than that of the control group, confirming that SOST increases under high glucose conditions (FIG. 1a). In addition, after treatment with 10 nM, 30 nM, and 50 nM siRNA (denoted as si10+HG, si30+HG, and si50+HG, respectively), SOST expression in the HG sample was shown to decrease in a dose-dependent manner.

When treated with VEGF, a higher level of SOST expression was observed compared to hyperglycemic condition, and the dose-dependent decreasing tendency of siRNA was also found to be higher (FIG. 1b).

In addition, the expression level of SOST upon SOST siRNA transfection was confirmed through immunofluorescence staining analysis.

As a result, after treatment with 30 nM siRNA, the SOST expression level was decreased. In addition, the immunostained values showed the expression of SOST in HRECs treated with HG and VEGF, regardless of whether SOST siRNA transfection was performed (FIG. 1c). According to these results, SOST expression was significantly increased after HG and VEGF treatment, but showed a markedly decreasing tendency after SOST knockdown.

In addition, the cytotoxic effect caused by SOST knockdown was confirmed. Specifically, the cytotoxic effect of SOST knockdown was investigated by examining the cell death effect after transfecting HRECs with 10 nM, 30 nM, and 50 nM of siRNA, regardless of HG treatment.

As a result, it was confirmed that almost 90% of the cells were still viable even after 24 hours of transfection (FIG. 1d). According to this, it was demonstrated that siRNA transfection did not induce cell death, given that the 30 mM HG-treated sample (siCON+HG) also showed 80% cell viability.

### Example 2. Confirmation of the effect of reducing hyperglycemia-induced inflammatory activation by SOST inhibition

It was confirmed whether inflammatory activation induced by hyperglycemia was suppressed by SOST inhibition. Specifically, it was analyzed whether the expression of cytokines in HRECs was decreased by silencing the SOST gene, and for this purpose, the expression of pro-inflammatory cytokines at the gene and protein levels was analyzed using qRT-PCR and WB.

As a result, contrary to the control sample (siCON), the expression of pro-inflammatory cytokines was increased in the sample treated with HG (siCON+HG). In contrast, the expression of all investigated gene markers was confirmed to decrease in a dose-dependent manner of SOST siRNA. Specifically, not only was the expression level of pro-inflammatory cytokines significantly downregulated from the sample transfected with the lowest concentration, 10 nM SOST siRNA (si10+HG), among the treatment concentrations, but such an effect was also confirmed to be at a statistically significant level (FIGs. 2a and 2b).

### Example 3. Confirmation of the effect of reducing hyperglycemia-induced Wnt signaling by SOST inhibition

Sclerostin, which is expressed by the SOST gene, is known as a Wnt antagonist. Since Wnt signaling is known to increase under the influence of hyperglycemia and induce retinal vascular dysfunction, an analysis was conducted to determine whether inhibition of the SOST gene could indirectly induce retinal vascular dysfunction in relation to Wnt signaling. Specifically, the effect of sclerostin/SOST knockdown on Wnt/β-catenin was confirmed through qRT-PCR and WB analysis after glucose treatment.

As a result, after silencing sclerostin, the gene and protein expression levels of β-catenin, Wnt3a, and Wnt5a in HRECs were shown to be decreased (FIGs. 3a and 3b). First, after HG treatment, the proteins related to Wnt signaling were shown to be expressed at higher levels, demonstrating that Wnt signaling is increased by hyperglycemia. In addition, when SOST, a Wnt antagonist, was inhibited, it was confirmed that the Wnt signaling increased by hyperglycemia was statistically significantly decreased.

Accordingly, according to these results, silencing SOST as a Wnt antagonist has minimal effect on Wnt, and therefore, it was demonstrated that inhibition of SOST does not induce retinal vascular dysfunction in relation to Wnt signaling, which is increased by hyperglycemia.

### Example 4. Confirmation of the effect of reducing VEGF-induced inflammatory activation by SOST inhibition

It was confirmed whether the expression levels of inflammatory cytokines occurring in HRECs after VEGF treatment could be reduced by inhibiting sclerostin through SOST inhibition. At this time, the gene and protein expression of inflammatory cytokines were analyzed using qRT-PCR and WB.

As a result (FIGs. 4a and 4b), all inflammatory cytokines showed a significant upregulation after VEGF treatment compared to the control group at both gene and protein levels. In addition, it was confirmed that all cytokine levels were downregulated in a dose-dependent manner of SOST/sclerostin knockdown by siRNA. In particular, as can be seen in the qRT-PCR and WB analysis, the expression of pro-inflammatory cytokines was greatly decreased in the samples treated with 30 nM and 50 nM SOST siRNA (si30+VEGF and si50+VEGF), and such an anti-inflammatory effect was confirmed to be at a statistically significant level.

### Example 5. Confirmation of the effect of SOST overexpression in HRECs

To confirm the effect of sclerostin overexpression on HRECs and pro-inflammatory cytokines, qRT-PCR, WB, and immunostaining analyses were performed. The study of SOST overexpression in HRECs was evaluated using various concentrations (10 ng, 30 ng, 50 ng, and 100 ng) of CRISPR-SOST knock-in.

As a result, SOST gene and protein expression increased according to the administered amount (FIG. 5a). The immunostaining analysis also showed that SOST was highly expressed compared to the control group when 100 ng CRISPR-SOST (SOST100) was treated (FIG. 5b). In addition, all inflammatory proteins including VEGFA and NF-κB were greatly increased after treatment with 100 ng SOST (FIG. 5c).

These results suggest that the presence of sclerostin in HRECs may induce the expression of inflammatory cytokines through the NF-κB pathway.

### Example 6. Confirmation of the effect of reducing oxidative stress by SOST inhibition

When oxidative stress was induced by treatment with high concentrations of glucose and VEGF, the green fluorescence expression of the oxidative stress marker increased, and when sclerostin expression was inhibited using siRNA, the expression of the oxidative stress marker decreased (FIG. 6a).

In addition, when sclerostin was overexpressed using CRISPR, the fluorescence expression of the oxidative stress marker increased (FIG. 6b).

### Example 7. Confirmation of the effect of reducing the expression of vascular endothelial growth factor and vascular adhesion markers by SOST inhibition

The effect on the expression of vascular endothelial growth factor and vascular adhesion markers was confirmed when SOST was inhibited. Specifically, the gene expression of the vascular adhesion markers VCAM-1 and ICAM-1, and vascular endothelial growth factor A (VEGFA) was analyzed after HG treatment or VEGF treatment, respectively, using qRT-PCR.

As a result, VEGFA, VCAM-1, and ICAM-1 were highly expressed after HG treatment or VEGF treatment, and were significantly downregulated after SOST knockdown (FIG. 7a). Decreased protein expression after SOST knockdown was detected, and the WB data of such protein expression were confirmed to correspond mostly with the gene expression data (FIG. 7b). In particular, when 30 and 50 nM of siRNA were transfected, the expression levels of ICAM-1 and VCAM-1 were shown to be almost undetectable.

Following Example 2, Example 4, and Example 6, it was demonstrated that HG and VEGF stimulate the expression of inflammatory cytokines, and that SOST silencing significantly reduces inflammatory expression. In addition, in the samples transfected with siRNA, sclerostin knockdown greatly reduced the expression not only of VEGFA, ICAM-1, and VCAM-1, but also of the protein transcription factor nuclear factor kappa B (NF-κB) and inducible NO synthase (iNOS). In contrast, higher expression was observed only in samples treated with HG and VEGF.

### Example 8. Confirmation of the effect of reducing angiogenesis by SOST inhibition

To evaluate the silencing effect of SOST siRNA on angiogenesis and vascular remodeling, pre-treated HRECs were seeded on Matrigel, and tube formation assay was performed

As a result (FIGs. 8a and 8b), intensive tube formation occurred in the siCON+HG and siCON+VEGF samples due to hyperglycemia. However, even under hyperglycemic conditions, SOST knockdown prevented tube formation. Specifically, in the si30+HG and si30+VEGF samples, angiogenesis was significantly inhibited by SOST siRNA treatment, and such effect was confirmed to be statistically significant.
In addition, in the SOST overexpression sample (SOST100) treated with 100 ng CRISPR-SOST, higher tube formation ability compared to the control group was observed (FIGs. 8c and 8d), thereby reconfirming that sclerostin expressed by SOST induces angiogenesis, and that when it is inhibited, tube formation is significantly suppressed.

The above description of the present invention is for illustrative purposes, and it will be understood by those skilled in the art that various modifications may be made in other specific forms without changing the technical spirit or essential characteristics of the present invention. Therefore, the embodiments described above should be understood as illustrative rather than limiting in all respects.

### INDUSTRIAL APPLICABILITY

According to pharmaceutical composition comprising a sclerostin inhibitor as an active ingredient for the prevention or treatment of diabetic eye disease, when sclerostin is inhibited, not only is the expression level of inflammatory cytokines significantly reduced under high glucose and VEGF treatment conditions, exhibiting excellent anti-inflammatory activity, but also a remarkable anti-angiogenic effect is observed due to the decreased expression of vascular endothelial growth factor and vascular adhesion markers, thereby confirming that inflammation and microvascular complications, which are the most significant features of diabetic eye diseases, can be suppressed. Furthermore, since oxidative stress caused by diabetes is reduced and cell death is not induced, it can be usefully applied as a preventive or therapeutic agent for diabetic eye diseases, and thus industrial applicability is recognized.

## Claims

1. A pharmaceutical composition comprising a sclerostin inhibitor as an active ingredient for prevention or treatment of diabetic eye disease.

2. The pharmaceutical composition according to claim 1, wherein the inhibitor is at least one of an expression inhibitor or an activity inhibitor.

3. The pharmaceutical composition according to claim 1 or claim 2, wherein the expression inhibitor or the activity inhibitor is at least one selected from the group consisting of small interfering RNA (siRNA), short hairpin RNA (shRNA), micro RNA (miRNA), antisense oligonucleotides (ASO), Clustered Regularly Interspaced Short Palindromic Repeats (CRISPR/Cas), natural products, proteins, peptidomimetics, antibodies, exosomes, and compounds.

4. The pharmaceutical composition in any one of claim 1 to claim 3, wherein the diabetic eye disease is at least one selected from the group consisting of diabetic retinopathy, diabetic macular edema, glaucoma, and cataracts.

5. The pharmaceutical composition in any one of claim 1 to claim 4, wherein the composition is any one formulation selected from the group consisting of suspensions, gels, ointments, injectable solutions, sprays, and eye drops.

6. The pharmaceutical composition in any one of claim 1 to claim 5, wherein the composition inhibits inflammatory responses and microvascular complications.

7. The pharmaceutical composition in any one of claim 1 to claim 6,
wherein the composition is **characterized by** one or more of the following:
inhibition of inflammatory cytokines within retinal cells;
inhibition of angiogenesis;
inhibition of vascular endothelial growth factor and vascular adhesion markers; and
reduction of oxidative stress.

8. A food composition comprising a sclerostin inhibitor as an active ingredient for prevention or improvement of diabetic eye disease.

9. The food composition according to claim 8, wherein the food is a health functional food.

10. A kit comprising a sclerostin inhibitor and instructions for prevention or treatment of diabetic eye disease.

11. A method for prevention or treatment of diabetic eye disease, comprising the step of administrating a composition comprising a sclerostin inhibitor as an active ingredient in a pharmaceutically effective amount to a subject in need thereof.

12. Use of a composition comprising a sclerostin inhibitor as an active ingredient for the prevention or treatment of diabetic eye disease.

13. Use of a composition comprising a sclerostin inhibitor as an active ingredient for preparing an agent for prevention or treatment of diabetic eye disease.
